# FASCICULE DE BREVET EUROPEEN

(11) **EP 2 440 878 B1**
(45) Date de publication et mention de la délivrance du brevet: **24.05.2017**
(21) Numéro de dépôt: 10734223.0
(22) Date de dépôt: 03.06.2010
(51) Int. Cl.: F41G 3/16, A61F 9/08, G06K 9/32, G06K 9/46, G09B 21/00

(54) **AIDE À LA VISÉE, POUR DES COMPÉTITIONS SPORTIVES, DE PERSONNES MAL VOYANTES OU NON VOYANTES**
ZIELHILFE FÜR SPORTWETTBEWERBE FÜR SEHBEHINDERTE ODER BLINDE PERSONEN
AIMING ASSISTANCE FOR SPORT COMPETITIONS FOR VISUALLY CHALLENGED OR BLIND PERSONS

(30) Priorité: 10.06.2009 FR 0953846
(43) Date de publication de la demande: 18.04.2012
(73) Titulaire: Electricité de France, 75008 Paris (FR)
(72) Inventeur: D'URSO, Guy, F-78800 Houilles (FR); HENAULT, Jean-Marie, F-78114 Magny les Hameaux (FR); CHANUSSOT, Jocelyn, F-38000 Grenoble (FR); COUTURIER-DOUX, Vincent, F-38700 La Tronche (FR)
(74) Mandataire: Cabinet Plasseraud
(86) Numéro de dépôt international: PCT/FR2010/051092
(87) Numéro de publication internationale: WO 2010/142891

(56) Documents cités:
- WO-A1-01/38823
- WO-A1-83/00919
- DE-A1- 10 253 646
- DE-A1- 10 360 716

## Description

La présente invention concerne une assistance automatisée pour des personnes handicapées visuelles (non voyantes ou mal voyantes) pour viser des cibles en tir à l'arc ou à la carabine, dans le cadre de compétitions sportives.

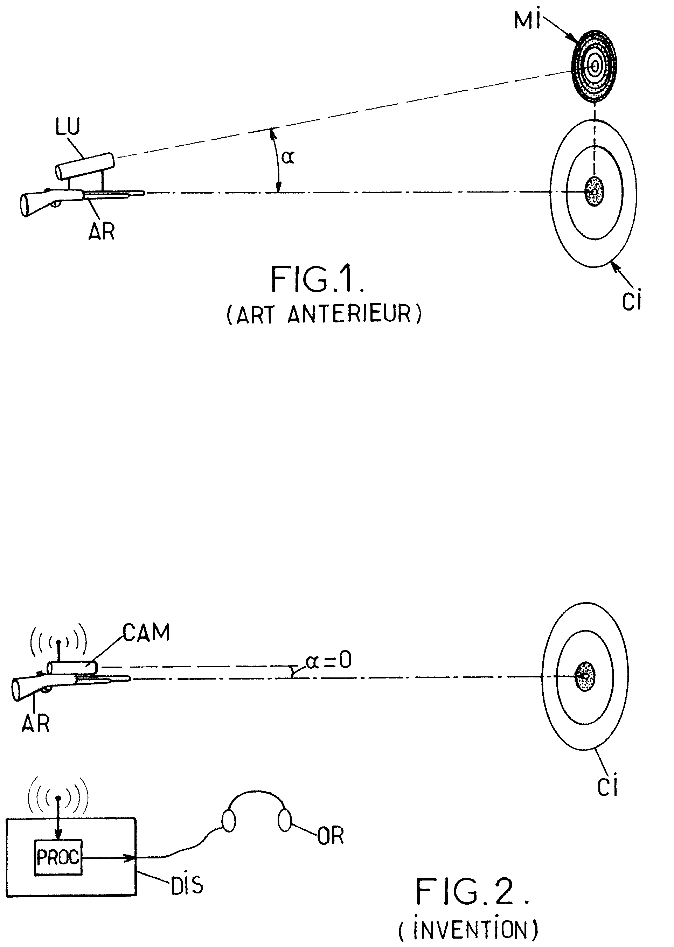

On a représenté, sur la figure 1 relative à l'art antérieur, un système d'aide à la visée, commercialisé par la société Swarovski, de droit allemand, et comportant une mire circulaire MI en niveaux de gris (allant du blanc au noir, depuis le centre de la mire jusqu'au bord), placée juste au-dessus de la cible CI. La mire est fortement éclairée par une lampe placée juste au-dessus et au foyer de la lunette LU montée sur l'arme AR se trouve un capteur de flux lumineux (typiquement un détecteur mono-pixel). Lorsque le centre de la mire MI est imagé sur ce détecteur, le flux est maximum, et un signal acoustique est envoyé au tireur pour lui indiquer que son arme vise bien le centre de la cible CI. On relèvera alors que la lunette LU est pré-orientée pour ce qui concerne l'angle α que forme l'axe passant par la lunette LU et la mire MI, relativement à l'axe de visée (de l'arme AR au centre de la cible CI).

Ainsi, le réglage de la position de la mire MI par rapport à la cible doit se faire avec précision, pour que la même valeur d'angle α soit respectée et que le tireur ait toujours bien son axe de visée en face du centre de la cible. En outre, la position de la mire MI par rapport à la cible CI doit être réglée à chaque fois en fonction de la position du tireur (en fonction de sa distance à la cible (200, 100 ou 50 mètres), le fait qu'il soit debout, accroupi ou couché, etc.).

Un tel système est alors assez lourd à utiliser et n'est pratiquement pas envisageable pour des compétitions.

En outre, un problème dit « de dévers » advient si le tireur ne garde pas exactement la même position lors du tir. En effet, ce type de système communique au tireur une direction de visée décalée par rapport à ce que le système cherche vraiment à détecter (ici, la mire MI), et, du fait de l'alignement initial de l'arme sur un point qui n'est pas le centre de la cible CI, il a été observé qu'un angle de devers α perturbait la qualité du tir.

Or, les cibles de tir pour compétition sont généralement homologuées, donc invariables en forme et/ou en couleur, et ce qu'elles soient destinées à être utilisées par des personnes handicapées ou par des personnes non handicapées. Il n'est donc pas question de modifier l'aspect des cibles pour les adapter aux systèmes d'aide à la visée existants.

Un dispositif similaire est divulgué dans la demande PCT WO 83/00919.

La présente invention vient alors améliorer la situation.

A cet effet, elle propose tout d'abord un procédé d'assistance à la visée d'une cible de tir, mise en oeuvre par des moyens informatiques, pour une personne mal voyante ou non voyante, selon la revendication 1. En cherchant à reconnaître la cible elle-même, par une reconnaissance de forme dans des modes de réalisation avantageux qui seront décrits plus loin, la pose d'une mire supplémentaire à repérer n'est plus nécessaire et aucun angle de dévers ne trouble la qualité du tir. On s'appuie en particulier sur des caractéristiques de la cible elle-même pour la reconnaître et repérer son centre.

Ainsi, dans une réalisation avantageuse, suite à l'identification de la cible dans les images filmées, le centre de la cible est déterminé et l'émission du signal perçu guide la personne dans l'orientation de l'arme de tir, en particulier vers le centre de la cible.

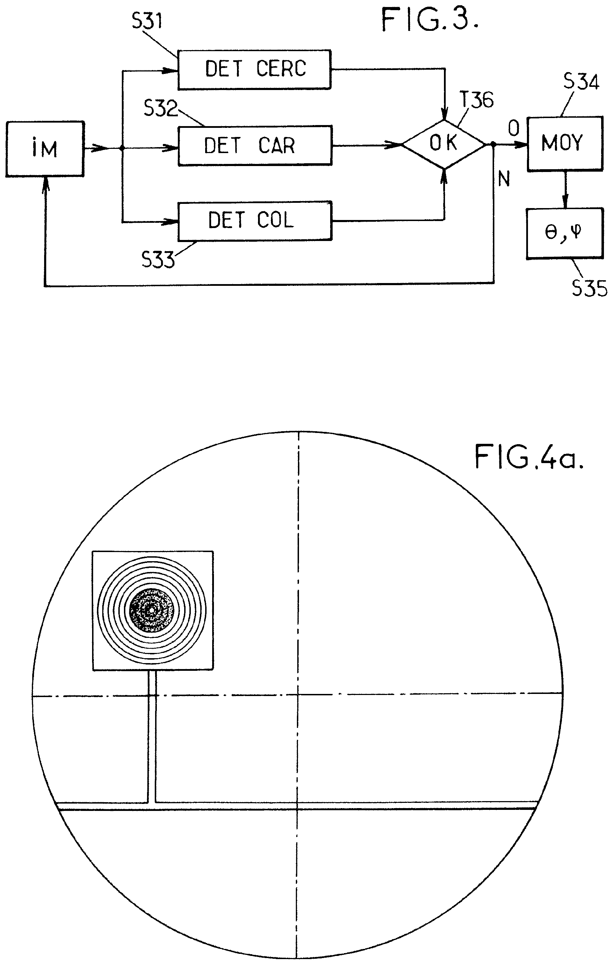

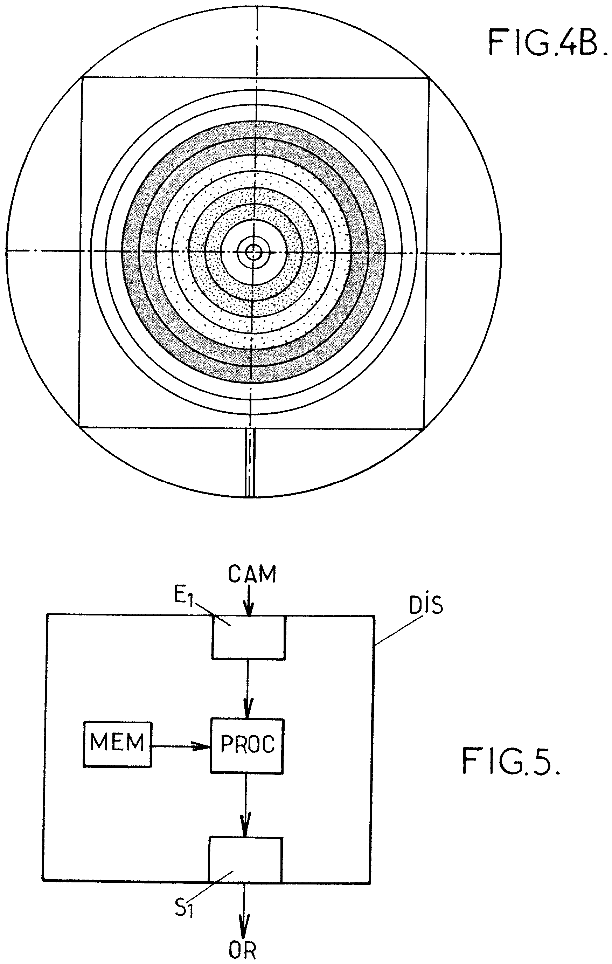

Cette réalisation avantageuse se fonde sur le fait que la cible présente un tracé (au moins un) selon un cercle, un disque ou une couronne circulaire, comme la plupart des cibles de tir comme on le verra plus loin en référence aux figures 4A et 4B. Ainsi, la reconnaissance de forme au sens de l'invention comporte, dans une réalisation avantageuse, une transformation de Hough pour identifier un tel tracé dans les images filmées, et, de là, le centre de ce tracé circulaire.

Plus précisément, si la cible présente une pluralité de tracés selon au moins un cercle, une couronne ou un disque concentriques, la transformation de Hough permet d'identifier, parmi cette pluralité de tracés circulaires, au moins deux tracés dont les centres respectifs sont séparés d'une distance inférieure à un seuil de tolérance choisi. Si ces deux tracés peuvent être identifiés, il peut être décidé que le centre de la cible est le barycentre des centres des deux tracés circulaires.

Avantageusement, on cherche à confirmer l'identification de la cible obtenue par la reconnaissance de forme précitée.

Dans une réalisation, on s'appuie sur un traitement utilisant la couleur de la cible pour confirmer la reconnaissance de forme. On peut par exemple réaliser une reconnaissance d'un contraste de couleurs entre différentes couronnes ayant chacune une couleur particulière, comme dans une cible de tir à l'arc. On peut aussi, en complément ou en variante, effectuer deux reconnaissances de forme :
- une première reconnaissance de forme sur les images, en niveaux de gris, et
- une deuxième reconnaissance de forme, utilisant la couleur, effectuée sur les images en couleur et en utilisant une composante de couleur choisie (vert par exemple dans une image RVB, pour « Rouge Vert Bleu »).

La deuxième reconnaissance de forme (sur l'image en couleur) vient alors confirmer le résultat de la première reconnaissance de forme en niveaux de gris.

Encore en complément ou en variante de la reconnaissance de forme des types décrits ci-avant, comme on prévoit habituellement un panneau de forme rectangulaire et comportant la cible à viser, la reconnaissance de forme de l'invention peut comporter l'identification de quatre coins à angles proches de 90° et espacés de distances relatives prédéterminées (par exemple d'une même distance relative, s'il s'agit d'un carré, comme on le verra plus loin).

Une réalisation particulière peut consister à :
- appliquer une première reconnaissance de forme comportant une transformation de Hough, et
- confirmer les résultats obtenus par une deuxième reconnaissance de forme basée, elle, sur l'identification des quatre coins.

Dans une réalisation concrète, on pourra prévoir un module de traitement relié à la caméra et grâce auquel :
- on stocke en mémoire des caractéristiques choisies de formes d'un modèle de cible,
- on reçoit de la caméra des données d'image courante,
- on recherche, dans les données d'image courante, des caractéristiques de formes, homologues des caractéristiques du modèle (par exemple un nombre donné de cercles concentriques, quatre coins avec des rapports prédéterminés de distances entre coins, ou autres), et
- en cas d'identification, dans les données d'image courante, de caractéristiques identiques aux caractéristiques du modèle, à une tolérance près (cette tolérance étant fonction par exemple de la prise en compte d'effets de perspective, de différence de températures de couleur d'éclairage, de la distance entre la caméra et la cible, ou autres) :
   - on décide de valider la reconnaissance de la cible dans l'image courante,
   - on détermine une distance entre la cible et un centre de visée de la caméra, et
   - on délivre un signal de sortie dont au moins un paramètre est fonction de cette distance.

En pratique, on délivre un signal qui est fonction :
- de la distance en hauteur (angle d'élévation à corriger pour le tireur), et
- de la distance horizontale à la cible (angle d'azimut à corriger pour le tireur).

On pourra choisir alors un signal de sortie acoustique et moduler ce signal avec au moins un paramètre parmi une amplitude, une amplitude de spatialisation (entre l'oreillette droite et l'oreillette gauche d'un casque à écouteurs, par exemple), une fréquence fondamentale et une fréquence de modulation (sous la forme par exemple de bips plus ou moins rapprochés), en fonction de la distance précitée.

La présente invention vise aussi un système d'assistance à la visée d'une cible de tir, pour la mise en oeuvre du procédé ci-avant, comportant :
- une caméra destinée à être placée sur une arme à disposition de la personne pour filmer une cible à viser,
- un dispositif relié à la caméra, d'identification de la cible par reconnaissance de forme dans des images que filme la caméra, et
- des moyens, par exemple un casque stéréophonique ou un écouteur, reliés au dispositif pour émettre un signal destiné à être perçu par la personne pour guider la personne dans l'orientation de l'arme de tir vers la cible.

D'autres caractéristiques et avantages de l'invention apparaîtront à l'examen de la description détaillée ci-après, et des dessins annexés sur lesquels, outre la figure 1 relative à l'art antérieur et décrite ci-avant :
- la figure 2 illustre schématiquement un équipement au sens de l'invention, monté sur une arme de tir,
- la figure 3 illustre les étapes globales d'un procédé au sens de l'invention pour l'assistance à la visée,
- les figures 4A et 4B sont des images de cibles prises avec une caméra dans deux configurations de focale distinctes, et
- la figure 5 représente schématiquement un dispositif au sens de l'invention pour traiter les données d'image que fournit la caméra, déterminer la présence ou non de la cible sur les images fournies, et en particulier y identifier, le cas échéant, le centre de la cible.

On se réfère à la figure 2 sur la laquelle un système d'aide à la visée au sens de l'invention comporte une caméra CAM filmant la cible CI pour identifier la cible par reconnaissance de forme lorsque l'arme (arc, carabine, fléchette ou autre) est pointée sur la cible. Lorsque la forme complète de la cible est reconnue par la caméra selon un zoom adapté à la distance présumée de la cible, un signal sonore retentit dans des oreillettes OR que porte la personne à assister, pour lui indiquer que l'arme AR est bien pointée sur la cible.

Dans un exemple de réalisation, la caméra CAM peut envoyer un signal vidéo par liaison filaire ou sans fil (Wifi ou autre) à un dispositif DIS que peut porter le tireur sur lui (par exemple dans un sac à dos ou autre). L'intérêt d'une telle mise en oeuvre est de limiter le poids du montage sur l'arme seulement au poids de la caméra car, bien entendu, plus l'arme est lourde et plus la fatigue du tireur rend son tir imprécis.

Un processeur PROC équipé d'une mémoire de travail reçoit les données vidéo, en définit une image et compare la forme de cette image à un modèle préenregistré (ou plus précisément, comme on le verra plus loin, compare certaines caractéristiques de cette image à celles du modèle). Dans un exemple de réalisation décrit en détail plus loin, la reconnaissance de forme s'appuie sur une transformation de Hough. Ce type de traitement est bien adapté pour la reconnaissance de formes circulaires. On rappelle que la reconnaissance d'un cercle par transformation de Hough comporte globalement les étapes suivantes :
- identifier des zones de contraste sensiblement uniforme dans une image (par exemple une zone où un niveau de gris est constant à une tolérance près),
- appliquer des tangentes à cette zone,
- déterminer les normales aux tangentes, et
- vérifier qu'elles sont (pratiquement) toutes sécantes (à une tolérance près) en un point qui est le centre d'un cercle ou d'un disque que forme la zone.

Cette détection de cercle, ainsi qu'une détection de carrés pour identifier les bords physiques de la cible, seront détaillées plus loin.

Les oreillettes OR peuvent se présenter sous la forme d'un casque à mini haut-parleurs respectifs pour l'oreille droite et l'oreille gauche du tireur. L'ajustement de l'angle d'élévation pour la visée peut s'effectuer par une variation de ton par rapport à une fréquence de référence. Ainsi, les mini haut-parleurs peuvent restituer :
- un premier signal variable en fréquence et dépendant de la hauteur de l'axe de visée par rapport à la cible, et
- un deuxième signal, de référence, de fréquence constante.

Ce deuxième signal peut être restitué à niveau sonore égal entre les deux mini haut-parleurs, de sorte que le tireur a la sensation que ce signal de référence est perçu « au milieu » entre ses deux oreilles. Le premier signal peut, quant à lui, être restitué à un niveau sonore variable entre les deux mini haut-parleurs, en fonction de l'angle d'azimut de l'axe de visée par rapport à la cible. Ainsi, lorsque le tireur a la sensation de faire en sorte, en manipulant son arme, qu'à l'écoute, les deux signaux semblent parfaitement « superposés », l'axe de visée doit traverser le centre de la cible et l'utilisateur peut tirer.

En variante, pour l'assistance de l'ajustement de l'angle d'azimut, il peut être prévu que le premier signal consiste en l'émission de bips répétés dont la fréquence augmente au fur et à mesure que l'axe de visée se rapproche de la cible, en azimut, jusqu'à un signal continu, auquel cas l'utilisateur peut tirer. On relèvera qu'un casque stéréophonique n'est pas utile dans cette variante et qu'une seule oreillette suffit.

Dans une variante moins sophistiquée mais qui a fourni néanmoins de bons résultats, le premier signal consiste en l'émission de bips répétés avec une fréquence qui augmente au fur et à mesure que l'axe de visée se rapproche de la cible, à la fois et indistinctement en azimut et en élévation, jusqu'à un signal continu, auquel cas l'utilisateur peut tirer. Ainsi, dans cette réalisation, la restitution d'un signal de référence n'est pas nécessaire et une seule oreillette suffit.

Toutefois, une difficulté est survenue dans la mise en oeuvre de l'invention, en particulier dans des conditions de plein air et de légère brume : augmenter la tolérance de détection de la cible notamment dans de telles conditions (avec des variations d'éclairement naturel de la cible, éventuellement une légère brume ou brouillard, etc.) permet certes de mieux détecter la cible mais entraîne un risque de détection de faux positifs, ce qui représente bien entendu un danger dans le contexte de tirs avec des armes.

Il a été proposé alors, dans une réalisation avantageuse, d'assortir la reconnaissance de forme précitée d'une reconnaissance basée sur la couleur de certains éléments de la cible, et/ou d'une reconnaissance de carrés, comme décrit plus loin.

Pour le tir à l'arc, les cibles ou « blasons » ont un diamètre extérieur de 122 cm et sont composés de cercles concentriques de couleur blanche, noire, bleue, rouge et jaune de l'extérieur vers l'intérieur, sur carton à fond blanc.

Pour le tir à la carabine, les cibles ont un diamètre extérieur de 155 mm composées de dix cercles concentriques comportant un visuel central (trois disques noirs concentriques) de 59,5 mm de diamètre, sur un carton de 170 mm de côté sur fond blanc.

Dans les deux cas, la détection de la cible et de son centre s'effectue, en référence à la figure 3, grâce à un traitement image par image. Le traitement comporte tout d'abord la détection de cercles en niveaux de gris (étape S31) dans une image courante IM. Les cercles correspondent :
- pour le tir à l'arc aux différents cercles concentriques de la cible de différentes couleurs, et
- pour le tir à la carabine, au visuel noir central.

La détection des cercles repose avantageusement sur l'utilisation de la transformée de Hough correspondant à la détermination de l'intersection des droites perpendiculaires aux contours, comme expliqué précédemment. Les points de l'image ayant un grand nombre d'intersections de droite sont les centres de cercles. La transformée de Hough est classique en soi et peut être présente dans des bibliothèques d'outils traditionnels de calcul informatique.

En particulier, la détection de cercles en niveau de gris comporte les opérations suivantes, dans un exemple de réalisation :
- conversion de l'image couleur en niveau de gris,
- application d'un filtre gaussien (par exemple de 9 pixels sur 9) pour réduire le bruit et diminuer les fausses détections, et
- application de la transformée de Hough avec conservation uniquement du premier cercle détecté (typiquement le cercle dont le centre possède le plus grand nombre d'intersections).

Le traitement peut comporter en outre, par exemple, la détection de carrés, en particulier des bords extérieurs du carton de la cible. Cette détection (étape S32) repose sur l'utilisation d'un traitement spécifique défini de la façon suivante :
- détection des contours dans l'image,
- sélection des contours avec les critères suivants :
   o quatre côtés à identifier et seulement quatre,
   o le contour est fermé,
   ∘ l'angle formé par deux cotés consécutifs doit être de 90° (à une tolérance près du fait d'un léger effet de perspective possible, selon la position du tireur),
   ∘ l'aire du contour doit être comprise entre deux seuils (mini et maxi),
   ∘ le rapport entre le plus grand côté et le plus petit côté doit être proche de un avec une certaine tolérance.

Dans l'exemple représenté sur la figure 3, plusieurs critères sont alors utilisés pour détecter une cible de tir :
- détection d'un cercle dans une image en niveau de gris (étape S31),
- détection de carrés (étape S32), et
- détection d'un cercle dans une image correspondant à une composante dans l'espace des couleurs (étape S33).

Pour le choix de la couleur servant à la troisième détection, la détection dans la composante « Vert » de l'espace des couleurs RVB (pour Rouge - Vert - Bleu) s'est avérée la plus pertinente, en conditions de compétition. Les étapes principales de cette détection sont les suivantes :
- sélection de la composante Vert (par exemple des pixels verts à une tolérance près) de l'image couleur,
- application d'un filtre gaussien (par exemple 9x3) pour réduire le bruit et diminuer les fausses détections, et
- application de la transformée de Hough avec conservation uniquement du premier cercle détecté (cercle dont le centre possède le plus grand nombre d'intersections).

On comprendra ainsi que l'on recommence le traitement de détection de cercles, indépendamment de celui réalisé en niveaux de gris à l'étape précédente S31.

Ainsi, pour une image courante IM :
- on effectue une détection de cercles dans cette image en niveau de gris (S31),
- on effectue pour la même image une détection de carrés (S32),
- on effectue pour la même image une détection de cercles dans l'image en couleur (S33).

Ensuite, on détermine les coordonnées (x, y pour une image bidimensionnelle) :
- du centre d'un premier cercle obtenu à l'étape S31,
- du centre d'un carré obtenu à l'étape S31,
- et du centre d'un deuxième cercle obtenu à l'étape S33.

On valide ces coordonnées si la distance entre les centres, pris deux à deux, est toujours inférieure à un seuil donné (proche de 0). Sinon, les détections sont rejetées (flèche N en sortie du test T36 de la figure 3) et on réitère les détections sur une nouvelle image courante. Aucun signal sonore n'est émis pour le tireur tant que les coordonnées des centres détectés n'ont pas été validées.

Si les détections sont positives, le barycentre des centres validés est considéré comme le centre de la cible à l'étape S34. Par comparaison de la position de ce barycentre avec celle d'une zone centrale d'image (par exemple les 9x9 pixels au centre de l'image) :
- l'écart en pixels selon l'axe vertical y est compté et un signal sonore correspondant est émis pour le tireur (par exemple à une fréquence dépendant de cet écart pris en valeur positive ou négative), pour lui indiquer un écart d'angle d'élévation ϕ de la visée, et
- l'écart en pixels selon l'axe vertical x est compté et un signal sonore correspondant est émis pour le tireur (par exemple avec une différence d'intensité sur les mini haut-parleurs de son casque stéréophonique dépendant de cet écart pris en valeur positive ou négative), pour lui indiquer un écart d'angle d'azimut θ de la visée.

La combinaison de deux détections parmi les trois présentées précédemment s'est avérée suffisante pour s'assurer que la cible était bien repérée, sans faux positifs.

Les essais ont révélé toutefois que la détection combinée des cercles en niveaux de gris et des carrés permettait bien de réduire les fausses détections, mais, en contrepartie, avait pour conséquence une augmentation du temps de traitement, ce qui est dommageable sur la précision du tir car le tireur est en mouvement pendant ce temps de traitement.

Les deux détections de cercles S31 et S33 (sur une image en niveau de gris d'une part, et sur la même image en couleur, d'autre part), basées toutes deux sur une transformation de Hough a donné de bons résultats, tant sur la fiabilité de la détection que sur la vitesse de traitement en temps réel, avec une restitution sonore pour le tireur perçue comme étant instantanée en fonction de ses mouvements.

Un autre paramètre qu'il convient d'optimiser est le réglage de la focale (ou « zoom ») de la caméra par rapport à la distance entre le tireur et la cible. D'une part, pour assurer une détection précise du centre de la cible sur l'image, les dimensions de la cible sur l'image courante doivent préférentiellement être les plus grandes possibles sans dépasser les bords d'image. Les coins de la cible devraient alors pratiquement coïncider avec les bords de l'image (comme le représente la vue de la figure 4B pour une cible homologuée de tir à l'arc). D'autre part, toutefois, pour que la signalisation sonore au tireur soit efficace, l'image doit englober la cible mais aussi ses alentours. En d'autres termes, pour que la caméra puisse attraper la cible dans son champ et que le tireur soit alors prévenu de limiter l'amplitude de ses mouvements, l'image que filme la caméra doit présenter typiquement une largeur de trois à cinq sept fois celle de la cible (comme le représente la vue de la figure 4A pour une cible homologuée de tir au pistolet à 10 mètres).

Bien entendu, selon les moyens à disposition et notamment la résolution en pixels de la caméra, il pourra être choisi un zoom plus ou moins puissant (dans le sens des faibles zooms lorsque la résolution de la caméra est élevée).

Pour utiliser une arme équipée d'une même caméra, de résolution donnée, dans plusieurs types de compétition, il est avantageux alors de prérégler le zoom de la caméra en fonction :
- de la taille de la cible, et
- de la distance de la cible,
pour chaque type de compétition.

Bien entendu, la présente invention ne se limite pas à la forme de réalisation décrite ci-avant à titre d'exemple ; elle s'étend à d'autres variantes.

On comprendra par exemple que le choix de la couleur verte pour la confirmation de la reconnaissance de forme réalisée sur l'image en couleur peut varier selon les conditions de mise en oeuvre et notamment selon les conditions d'éclairage de la cible (en salle ou en plein air par exemple).

Par ailleurs, l'utilisation de la couleur dans les images obtenues peut impliquer une reconnaissance de forme dans une couleur donnée (le vert par exemple), mais aussi une reconnaissance de contraste de couleur dans la cible. Cette réalisation peut être mise en oeuvre notamment dans le cas d'une cible de tir à l'arc où il est possible de s'appuyer sur une reconnaissance des couleurs des différentes couronnes circulaires concentriques pour confirmer la reconnaissance de forme en niveaux de gris (figure 4B).

La présente invention vise aussi, en référence à la figure 5, un dispositif DIS d'un système d'assistance à la visée d'une cible de tir, pour une personne mal voyante ou non voyante, comportant :
- une mémoire MEM pour stocker des caractéristiques choisies de formes d'un modèle de cible,
- une entrée E1 pour recevoir de la caméra CAM des données d'image courante, et
- un processeur PROC pour :
   - rechercher, dans les données d'image courante, des caractéristiques de formes, homologues des caractéristiques du modèle, et
   - en cas d'identification, dans les données d'image courante, de caractéristiques identiques aux caractéristiques du modèle, à une tolérance près :
      ∘ décider une reconnaissance de la cible dans l'image courante,
      ∘ déterminer une distance entre la cible et un centre de visée de la caméra,
- et une sortie S1 pour délivrer un signal dont au moins un paramètre est fonction de cette distance.

La présente invention vise aussi un programme informatique destiné à être stocké en mémoire d'un tel dispositif et comportant des instructions pour la mise en oeuvre du procédé selon l'invention, lorsqu'elles sont exécutées par le processeur PROC du dispositif. A titre d'exemple, la figure 3 peut représenter un organigramme d'un tel programme informatique.

## Revendications

1. Procédé d'assistance à la visée d'une cible de tir, pour une personne mal voyante ou non voyante, ledit procédé comportant :
- l'équipement d'une arme de tir (AR), à disposition de la personne, par une caméra (CAM) placée sur l'arme pour filmer la cible à viser, le
- une identification de la cible et une détermination d'une distance entre le centre de la cible et un centre de visée de la caméra par reconnaissance de forme dans des images que filme la caméra (S31, S32, S33),
- l'émission d'un signal, dont au moins un paramètre est fonction de ladite distance, perçu par la personne pour guider la personne dans l'orientation de l'arme de tir vers la cible,
dans lequel :
- la cible présente au moins un tracé selon un cercle, un disque ou une couronne circulaire,
- la reconnaissance de forme comporte une transformation de Hough pour identifier ledit tracé dans les images filmées.

2. Procédé selon la revendication 1, **caractérisé en ce que**, suite à l'identification de la cible dans les images filmées, le centre de la cible est déterminé et l'émission du signal perçu guide la personne dans l'orientation (S35) de l'arme de tir vers le centre de la cible.

3. Procédé selon l'une des revendications 1 et 2, dans lequel la cible présente une pluralité de tracés selon au moins un cercle, une couronne ou un disque concentriques, **caractérisé en ce que** la transformation de Hough identifie, parmi ladite pluralité de tracés, au moins deux tracés dont les centres respectifs sont séparés d'une distance inférieure à un seuil de tolérance choisi.

4. Procédé selon l'une des revendications précédentes, **caractérisé en ce que** l'on prévoit un traitement utilisant la couleur de la cible (S33) pour confirmer la reconnaissance de forme.

5. Procédé selon l'une la revendication 4, **caractérisé en ce que** l'on applique au moins :
- une première reconnaissance de forme (S31) sur les images, en niveaux de gris, et
- une deuxième reconnaissance de forme (S33) sur les images, en couleur, en utilisant une composante de couleur choisie.

6. Procédé selon l'une des revendications précédentes, dans lequel un panneau de forme rectangulaire comporte la cible à viser, **caractérisé en ce que** la reconnaissance de forme (S32) comporte l'identification de quatre coins à angles proches de 90° et espacés de distances relatives prédéterminées.

7. Procédé selon les revendications précédentes, **caractérisé en ce que** l'on applique au moins :
- une première reconnaissance de forme (S31) comportant une transformation de Hough, et
- une deuxième reconnaissance de forme (S32) basée sur l'identification des quatre coins.

8. Procédé selon l'une des revendications précédentes, **caractérisé en ce que** :
- on stocke en mémoire (MEM) des caractéristiques choisies de formes d'un modèle de cible,
- on reçoit de la caméra (CAM) des données d'image courante,
- on recherche (S31, S32, S33), dans les données d'image courante, des caractéristiques de formes, homologues des caractéristiques du modèle, et
- en cas d'identification, dans les données d'image courante, de caractéristiques identiques aux caractéristiques du modèle, à une tolérance près :
• la cible est reconnue dans l'image courante (T36),
• on détermine une distance (S35) entre la cible et un centre de visée de la caméra, et
• on délivre un signal de sortie dont au moins un paramètre est fonction de ladite distance.

9. Procédé selon la revendication 8, **caractérisé en ce que** le signal de sortie est acoustique et ledit paramètre est choisi parmi une amplitude, une amplitude de spatialisation, une fréquence fondamentale et une fréquence de modulation, en fonction de ladite distance.

10. Système d'assistance à la visée d'une cible de tir, pour une personne mal voyante ou non voyante, ledit système comportant :
- une caméra (CAM) configuré pour être placée sur une arme à disposition de la personne pour filmer une cible à viser,
- un dispositif (DIS) relié à la caméra, d'identification de la cible par reconnaissance de forme dans des images que filme la caméra, et
- des moyens (OR) reliés au dispositif (DIS) configurés pour émettre un signal destiné à être perçu par la personne pour guider la personne dans l'orientation de l'arme de tir vers la cible,
ledit système étant configuré de manière à mettre en oeuvre le procédé selon l'une quelconque des revendications 1 à 9.

11. Système selon la revendication 10, **caractérisé en ce que** le dispositif (DIS) comprend:
- une mémoire (MEM) configurée pour stocker des caractéristiques choisies de formes d'un modèle de cible,
- une entrée (E1) configurée pour recevoir de la caméra des données d'image courante, et
- un processeur (PROC) configuré pour :
• rechercher, dans les données d'image courante, des caractéristiques de formes, homologues des caractéristiques du modèle, et
• en cas d'identification, dans les données d'image courante, de caractéristiques identiques aux caractéristiques du modèle, à une tolérance près :
∘ décider une reconnaissance de la cible dans l'image courante,
∘ déterminer une distance entre le centre de la cible et un centre de visée de la caméra,
- et une sortie (S1) configurée pour délivrer un signal dont au moins un paramètre est fonction de ladite distance.

12. Programme informatique stocké en mémoire d'un dispositif (DIS) du système selon la revendication 11, comportant des instructions pour la mise en oeuvre d'un procédé d'assistance à la visée d'une cible de tir, pour une personne mal voyante ou non voyante, comportant :
- une identification de la cible et une détermination d'une distance entre le centre de la cible et un centre de visée de la caméra par reconnaissance de forme dans des images que filme la caméra (S31, S32, S33),
- l'élaboration d'un signal dont au moins un paramètre est fonction de ladite distance,
- l'émission d'un signal, dont au moins un paramètre est fonction de ladite distance, perçu par la personne pour guider la personne dans l'orientation de l'arme de tir vers la cible,
et dans lequel la cible présente au moins un tracé selon un cercle, un disque ou une couronne circulaire, la reconnaissance de forme comportant une transformation de Hough pour identifier ledit tracé dans les images filmées,
lorsque lesdites instructions sont exécutées par le processeur (PROC) du dispositif (DIS).

## Patentansprüche

1. Verfahren zur Unterstützung beim Zielen auf ein Schussziel für eine sehbehinderte oder blinde Person, wobei das Verfahren Folgendes umfasst:
- die Ausrüstung einer Schusswaffe (AR) mit einer für die Person verfügbaren Kamera (CAM), die auf der Waffe platziert wird, um das anzuvisierende Ziel aufzunehmen,
- eine Identifizierung des Ziels und eine Bestimmung eines Abstandes zwischen dem Zentrum des Ziels und einem Zentrum des Sichtfeldes der Kamera durch Formerkennung in Bildern, die die Kamera aufnimmt (S31, S32, S33),
- die Aussendung eines Signals, von dem mindestens ein Parameter eine Funktion des Abstandes ist, das von der Person wahrgenommen wird, um die Person bei der Ausrichtung der Schusswaffe auf das Ziel zu leiten,
wobei:
- das Ziel mindestens eine Linie gemäß einem Kreis, einer kreisförmigen Scheibe oder einem kreisförmigen Kranz aufweist,
- die Formerkennung eine Hough-Transformation umfasst, um die Linie in den aufgenommenen Bildern zu erkennen.

2. Verfahren nach Anspruch 1, **dadurch gekennzeichnet, dass** nach der Identifizierung des Ziels in den aufgenommenen Bildern das Zentrum des Ziels bestimmt wird und durch die Aussendung des wahrgenommenen Signals die Person bei der Ausrichtung (S35) der Schusswaffe auf das Zentrum des Ziels geleitet wird.

3. Verfahren nach einem der Ansprüche 1 und 2, wobei das Ziel eine Vielzahl konzentrischer Linien gemäß mindestens einem Kreis, einem Kranz oder einer Scheibe aufweist, **dadurch gekennzeichnet, dass** durch die Hough-Transformation unter der Vielzahl der Linien mindestens zwei Linien identifiziert werden, deren jeweilige Zentren durch einen kleineren Abstand als einer gewählten Toleranzschwelle getrennt sind.

4. Verfahren nach einem der vorhergehenden Ansprüche, **dadurch gekennzeichnet, dass** eine Verarbeitung vorgesehen ist, bei der die Farbe des Ziels (S33) verwendet wird, um die Formerkennung zu bestätigen.

5. Verfahren nach Anspruch 4, **dadurch gekennzeichnet dass** mindestens Folgendes angewandt wird:
- eine erste Formerkennung (S31) auf den Bildern in Graustufen und
- eine zweite Formerkennung (S33) auf den Bildern in Farbe unter Verwendung einer gewählten Farbkomponente.

6. Verfahren nach einem der vorhergehenden Ansprüche, wobei ein Schild in Rechteckform das anzuvisierende Ziel umfasst, **dadurch gekennzeichnet, dass** die Formerkennung (S32) die Identifizierung von vier Ecken mit Winkeln nahe 90° und mit vorbestimmten relativen Abständen beabstandet umfasst.

7. Verfahren nach den vorhergehenden Ansprüchen, **dadurch gekennzeichnet, dass** wenigstens:
- eine erste Formerkennung (S31), die eine Hough-Transformation umfasst, und
- eine zweite Formerkennung (S32) basierend auf der Identifizierung der vier Ecken
angewandt wird.

8. Verfahren nach einem der vorhergehenden Ansprüche, **dadurch gekennzeichnet, dass**:
- im Speicher (MEM) gewählte Eigenschaften von Formen eines Zielmodells gespeichert werden,
- von der Kamera (CAM) Daten des aktuellen Bildes empfangen werden,
- in den Daten des aktuellen Bildes mit den Eigenschaften des Modells übereinstimmende Eigenschaften von Formen gesucht (S31, S32, S33) werden und
- bei Identifizierung von mit dem Modell identischen Eigenschaften in den Daten des aktuellen Bildes bis auf eine Toleranz:
• das Ziel im aktuellen Bild erkannt wird (T36),
• ein Abstand zwischen dem Ziel und einem Zentrum des Sichtfeldes der Kamera bestimmt wird (S35), und
• ein Ausgangssignal geliefert wird, von dem mindestens ein Parameter eine Funktion des Abstandes ist.

9. Verfahren nach Anspruch 8, **dadurch gekennzeichnet, dass** das Ausgangssignal akustisch ist und der Parameter aus einer Amplitude, einer Spatialisierungsamplitude, einer Grundfrequenz und einer Modulationsfrequenz als Funktion des Abstandes ausgewählt wird.

10. System zur Unterstützung beim Zielen auf ein Schussziel für eine sehbehinderte oder blinde Person, wobei das System Folgendes umfasst:
- eine Kamera (CAM), die dafür konfiguriert ist, um für eine Person verfügbar auf einer Waffe platziert zu werden, um ein anzuvisierendes Ziel aufzunehmen,
- eine mit der Kamera verbundene Vorrichtung (DIS) zum Identifizieren des Ziels durch Formerkennung in Bildern, die die Kamera aufnimmt, und
- Mittel (OR), verbunden mit der Vorrichtung (DIS), die dafür konfiguriert sind, ein Signal auszusenden, das dazu bestimmt ist, von der Person wahrgenommen zu werden, um die Person bei der Ausrichtung der Schusswaffe auf das Ziel zu leiten, wobei das System dafür konfiguriert ist, das Verfahren nach einem der Ansprüche 1 bis 9 durchzuführen.

11. System nach Anspruch 10, **dadurch gekennzeichnet, dass** die Vorrichtung (DIS) Folgendes umfasst:
- einen Speicher (MEM), der dafür konfiguriert ist, gewählte Eigenschaften von Formen eines Zielmodells zu speichern,
- einen Eingang (E1), der dafür konfiguriert ist, von der Kamera Daten des aktuellen Bildes zu empfangen, und
- einen Prozessor (PROC), der dafür konfiguriert ist:
• in den Daten des aktuellen Bildes mit den Eigenschaften des Modells übereinstimmende Eigenschaften von Formen zu suchen und
• bei Identifizierung von mit dem Modell identischen Eigenschaften in den Daten des aktuellen Bildes bis auf eine Toleranz:
∘ zu entscheiden, dass das Ziel im aktuellen Bild erkannt ist,
∘ einen Abstand zwischen dem Zentrum des Ziels und dem Zentrum des Sichtfeldes der Kamera zu bestimmen,
- und einen Ausgang (S1), der dafür konfiguriert ist, ein Signal zu liefern, von dem mindestens ein Parameter eine Funktion des Abstandes ist.

12. Datenverarbeitungsprogramm, gespeichert im Speicher einer Vorrichtung (DIS) des Systems nach Anspruch 11, umfassend Anweisungen für die Durchführung eines Verfahrens zur Unterstützung beim Zielen auf ein Schussziel für eine sehbehinderte oder blinde Person, umfassend:
- eine Identifizierung des Ziels und eine Bestimmung eines Abstandes zwischen dem Zentrum des Ziels und einem Zentrum des Sichtfeldes der Kamera durch Formerkennung in Bildern, die die Kamera aufnimmt (S31, S32, S33),
- die Erzeugung eines Signals, von dem mindestens ein Parameter eine Funktion des Abstandes ist,
- die Aussendung eines Signals, von dem mindestens ein Parameter eine Funktion des Abstandes ist, das von der Person wahrgenommen wird, um die Person bei der Ausrichtung der Schusswaffe auf das Ziel zu leiten,
und wobei das Ziel mindestens eine Linie gemäß einem Kreis, einer kreisförmigen Scheibe oder einem kreisförmigen Kranz aufweist, wobei die Formerkennung eine Hough-Transformation umfasst, um die Linie in den aufgenommenen Bildern zu erkennen, wenn die Anweisungen durch den Prozessor (PROC) der Vorrichtung (DIS) ausgeführt werden.

## Claims

1. Method to assist a visually challenged or blind person with aiming at a shooting target, the method comprising:
- providing the shooting weapon (AR) to be used by the person with a camera (CAM) placed on the weapon to capture images of the target to be aimed at,
- identifying the target and determining a distance between the center of the target and a center of aim of the camera by shape recognition in the images captured by the camera (S31, S32, S33),
- emitting a signal, at least one parameter of which is a function of said distance, to be perceived by the person to guide the person in orienting the shooting weapon towards the target,
wherein:
- the target displays at least one shape which forms a circle, a disk, or a circular ring,
- shape recognition comprises a Hough transformation to identify said shape in the captured images.

2. Method according to claim 1, **characterized in that**, after the target is identified in the captured images, the center of the target is determined and the emitting of the signal to be perceived guides the person in orienting (S35) the shooting weapon towards the center of the target.

3. Method according to either of claims 1 or 2, wherein the target displays a plurality of shapes which form at least one concentric circle, ring, or disk, **characterized in that** the Hough transformation identifies among said plurality of shapes at least two shapes having respective centers separated by a distance of less than a chosen tolerance threshold.

4. Method according to any one of the above claims, **characterized in that** processing is applied which uses the color of the target (S33) to confirm the shape recognition.

5. Method according to claim 4, **characterized in that** at least the following are applied:
- a first shape recognition (S31), to the images as grayscale images, and
- a second shape recognition (S33), to the images as color images, using a selected color component.

6. Method according to any one of the above claims, wherein a rectangular board comprises the target to be aimed at, **characterized in that** the shape recognition (S32) comprises the identification of four corners having angles close to 90° and spaced apart by predetermined relative distances.

7. Method according to the preceding claims, **characterized in that** at least the following are applied:
- a first shape recognition (S31) comprising a Hough transformation, and
- a second shape recognition (S32) based on the identification of four corners.

8. Method according to any one of the above claims, **characterized in that**:
- selected shape characteristics of a target model are stored in memory (MEM),
- data for the current image are received from the camera (CAM),
- the current image data are examined for shape characteristics which are homologous to the model characteristics (S31, S32, S33), and
- if characteristics identical to the model characteristics are identified in the current image data, within a certain tolerance:
• the target is recognized in the current image (T36),
• a distance (S35) is determined between the target and a center of aim of the camera, and
• an output signal is issued for which at least one parameter is a function of said distance.

9. Method according to claim 8, **characterized in that** the output signal is acoustic and said parameter is chosen from among an amplitude, an amplitude of spatialization, a fundamental frequency, and a modulation frequency, as a function of said distance.

10. System to assist a visually challenged or blind person with aiming at a shooting target, said system comprising:
- a camera (CAM) configured to be placed on a weapon to be used by the person, to capture images of a target,
- a device (DIS) connected to the camera, for identifying the target by shape recognition in images captured by the camera, and
- a means (OR) connected to the device (DIS), configured to emit a signal to be perceived by the person to guide the person in orienting the shooting weapon towards the target,
said system being configured so that it may implement the method according to any one of claims 1 to 9.

11. System according to claim 11, **characterized in that** the device (DIS) comprises:
- a memory (MEM) configured to store the selected shape characteristics of a target model,
- an input (E1) configured to receive current image data from a camera, and
- a processor (PROC) configured to
• search the current image data for shape characteristics that are homologous to the model characteristics, and
• if characteristics identical to the model characteristics are identified in the current image data, within a certain tolerance:
∘ decide that the target is recognized in the current image,
∘ determine a distance between the center of the target and a center of aim of the camera,
- and an output (S1) configured to deliver a signal for which at least one parameter is a function of said distance.

12. Computer program stored in the memory of a device (DIS) of the system according to claim 11, comprising instructions for carrying out a method to assist a visually challenged or blind person with aiming at a shooting target, the method comprising:
- identifying the target and determining a distance between the center of the target and a center of aim of the camera by shape recognition in the images captured by the camera (S31, S32, S33),
- producing a signal, at least one parameter of which is a function of said distance,
- emitting a signal, at least one parameter of which is a function of said distance, to be perceived by the person to guide the person in orienting the shooting weapon towards the target,
and wherein the target displays at least one shape which forms a circle, a disk, or a circular ring, the shape recognition comprising a Hough transformation to identify said shape in the captured images,
when these instructions are executed by a processor (PROC) of the device (DIS).
